# EUROPEAN PATENT APPLICATION

(11) **EP 4 813 378 A1**
(43) Date of publication of application: **30.09.2026**
(21) Application number: 24884813.7
(22) Date of filing: 30.10.2024
(51) Int. Cl.: A61K 31/05, A61P 17/06, A61P 17/08, A61P 31/02

(54) **SOLUTION-TYPE PHARMACEUTICAL COMPOSITION OF PHENOL DERIVATIVE, AND FORMULATION AND USE THEREOF**

(30) Priority: 02.11.2023 CN 202311450168
(71) Applicant: Thederma Shanghai Co., Ltd., Shanghai 201203 (CN)
(72) Inventor: JIA, Jianmin, Shanghai 201203 (CN); MIAO, Pengfei, Shanghai 201203 (CN); CHEN, Ming, Shanghai 201203 (CN); MA, Zhilong, Shanghai 201203 (CN)
(74) Representative: Ipsilon
(86) International application number: PCT/CN2024/128475
(87) International publication number: WO 2025/092813

(57) **Abstract**

A solution-type pharmaceutical composition of a phenol derivative, and a formulation and use thereof. The pharmaceutical composition comprises the following components: an active ingredient, a humectant, a penetration enhancer, a chelating agent, a pH regulator, and a solvent; wherein the active ingredient is selected from at least one of a compound of formula (I) and a pharmaceutically acceptable salt thereof. The composition has good physical and chemical stability, is suitable for treating autoimmune inflammation, is particularly suitable for treating diseases described above with hair part onset, alleviates the problems of high medication difficulty and poor treatment effect of the hair part, and remarkably improves the medication compliance and life quality of patients.

## Description

The present application claims priority to Chinese Patent Application No. 202311450168.8 entitled "Solution-Type Pharmaceutical Composition of Phenol Derivative, and Formulation and Use Thereof" and filed with CNIPA on November 2, 2023, the disclosure of which is incorporated herein by reference in its entirety.

### TECHNICAL FIELD

The present disclosure belongs to the field of pharmaceutical compositions, and particularly relates to a solution-type pharmaceutical composition of a phenol derivative, and a formulation and use thereof.

### BACKGROUND

Autoimmune inflammatory diseases include skin diseases such as psoriasis, atopic dermatitis, and seborrheic dermatitis. Among them, psoriasis, commonly known as "silver scale disease," is a common chronic, inflammatory, and systemic disease induced by the interaction of multiple factors such as genetic factors, environmental factors, and immune factors. It has a long disease course, is difficult to treat, and is prone to recurrence. In some cases, it remains uncured for life, greatly affecting the physical health and mental condition of patients. Common sites of onset include the scalp, sacral region, extensor surfaces of the limbs, flexural surfaces, and skin folds. Nearly two-thirds of patients develop lesions in hair-bearing areas, especially the scalp, which is a predilection site for psoriasis. Existing studies have shown that scalp lesions are involved in 50% to 80% of psoriasis patients.

Seborrheic dermatitis (SD) is a chronic papulosquamous superficial inflammatory skin disease occurring in sebaceous gland-rich areas. Typical skin lesions are dark red or yellow-red patches formed by the fusion of follicular papules and are covered with greasy scales or crusts, may be accompanied by varying degrees of pruritus. SD is a chronic and recurrent inflammatory skin disease, usually presenting as red scaly eruptions. Its incidence in the general healthy population is 5%, among which 70.3% of cases occur on the head, and it is common in infants, adolescents, and adults. Seborrheic dermatitis is a scalp disorder that differs from simple dandruff in that it presents erythema as a sign of inflammation, more severe desquamation of the scalp, and is sometimes accompanied by itching and burning sensations, as well as eczema occurring on other parts of the body. The disease may occur in the form of plaques, but often affects the entire scalp and extends beyond the hairline, frequently involving the forehead, around the neck, and both ears. In severe patients, secondary infection may occur on the scalp, and the changes may manifest as a spongy texture, blistering, crust formation, and possible exudation. Seborrheic dermatitis also frequently occurs in infancy and usually resolves spontaneously at 8-12 months of age. Changes in the scalp of infants include erythema, desquamation of scalp, and occasionally vesicles and crusts. These changes may spontaneously resolve within weeks, recur intermittently, or persist throughout childhood. These changes are often accompanied by similar lesions around the eyelids, nose, and both ears. Thereafter, the condition often appears after puberty and may persist for life, sometimes even becoming more severe. About 1-3% of the population suffers from this disease. The etiology and pathogenesis of SD remain unclear, and the complex interaction among *Malassezia,* keratinocytes, and immune responses plays a crucial role in the development of SD. Seborrheic dermatitis shares some pathogenic factors with inflammatory skin diseases such as atopic dermatitis, including, for example, the regulation of factors that stimulate keratinocyte proliferation and differentiation, and skin barrier disruption. Atopic dermatitis usually presents a Th2 immune response with increased levels of interleukin (IL)-4 and IL-13; seborrheic dermatitis mainly involves Th2 and Th17 responses with increased levels of IL-4, IL-17, and IL-8.

Current treatment methods for seborrheic dermatitis include anti-inflammatory drugs alone, such as topical hormone preparations and calcineurin inhibitors, supplemented with skin-moisturizing skincare products; however, the efficacy is relatively low, and symptoms are prone to recur after drug withdrawal.

The treatment of psoriasis mainly aims at controlling symptoms and improving quality of life, including topical drugs, systemic drugs, and phototherapy. Topical drugs are first-line basic therapy and are suitable for more than 90% of patients. Topical formulations mainly include creams, ointments, gels, and the like. However, commonly used topical dosage forms such as creams, ointments, and gels, due to reasons such as the following: the scalp has dense and active sebaceous glands, and the bioavailability of lipophilic cream preparations is affected by sebaceous glands and hair follicles, resulting in poor therapeutic effects; furthermore, due to the obstruction of hair, these formulations are inevitably applied onto the hair during use, which not only causes waste of the drug, but is also often difficult to wash off, resulting in greasy hair appearance and unpleasant odor, leading to poor patient compliance. As a result, topical drugs are difficult to use in the hair-bearing disease area, and cannot be used effectively.

Therefore, providing a stable and/or safe pharmaceutical composition/formulation to improve the problems of difficulty in administration, poor therapeutic effect, and poor patient compliance in the treatment of the above diseases has long been a focus of efforts in this field.

### SUMMARY

**In** order to improve the above technical problems, the present disclosure provides a pharmaceutical composition, including the following components: an active ingredient, a humectant, a penetration enhancer, a chelating agent, a pH regulator, and a solvent, wherein the active ingredient is selected from at least one of the compound represented by Formula (I) and pharmaceutically acceptable salts thereof:

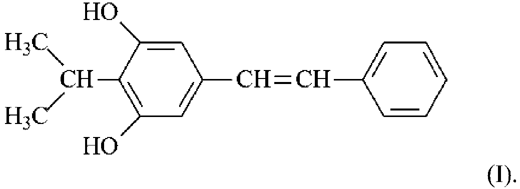

In another aspect, the present disclosure provides a pharmaceutical composition, including the following components: an active ingredient, a humectant, a penetration enhancer, a chelating agent, a thickener, a pH regulator, and a solvent, wherein the active ingredient is selected from at least one of the compound represented by Formula (I) and pharmaceutically acceptable salts thereof:

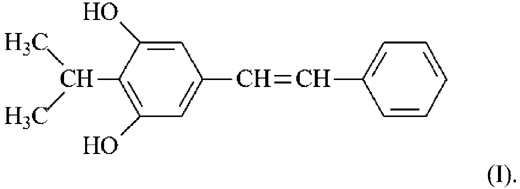

In some embodiments of the present disclosure, the pharmaceutical composition is a solution, preferably a solution-type pharmaceutical composition for spray or aerosol administration.

According to embodiments of the present disclosure, the compound represented by Formula (I) may be an E-type or Z-type isomer, preferably the E-type isomer represented by Formula (I-1) below:

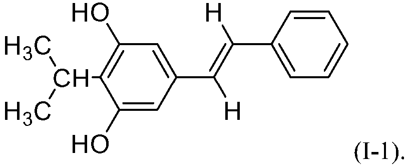

According to a preferred embodiment of the present disclosure, the compound represented by Formula (I-1) is Benvitimod.

According to embodiments of the present disclosure, the pharmaceutically acceptable salt may be selected from an addition salt of the compound of Formula (I) with an alkali.

In some preferred embodiments of the present disclosure, the pharmaceutical composition uses only the compound represented by Formula (I) as the active ingredient, and preferably uses only the E-type isomer shown in Formula (I-1) (i.e., Benvitimod) as the active ingredient.

According to an embodiment of the present disclosure, in the pharmaceutical composition, the active ingredient is 0.1-8 parts by weight, for example, 0.5-5 parts, and exemplarily 0.5 parts, 0.75 parts, 1 part, 1.5 parts, 2 parts, 3 parts, or 4 parts.

According to an embodiment of the present disclosure, the humectant is selected from one or more of propylene glycol, glycerol, and polyethylene glycol, and preferably propylene glycol.

According to an embodiment of the present disclosure, in the pharmaceutical composition, the humectant is 10-35 parts by weight, for example, 15-30 parts, and exemplarily 15 parts, 18 parts, 20 parts, 22 parts, 25 parts, 27 parts, or 30 parts.

According to an embodiment of the present disclosure, the penetration enhancer is selected from one or more of diethylene glycol monoethyl ether, ethanol, menthol, N-methylpyrrolidone, propylene carbonate, and dimethyl isosorbide, and preferably diethylene glycol monoethyl ether.

According to an embodiment of the present disclosure, in the pharmaceutical composition, the penetration enhancer is 10-50 parts by weight, for example, 15-45 parts, and exemplarily 20 parts, 22 parts, 25 parts, 27 parts, 30 parts, 35 parts, 40 parts, or 41 parts.

According to an embodiment of the present disclosure, the chelating agent is selected from a metal-ion-chelating agent, for example, selected from one or more of ethylenediaminetetraacetic acid, sodium gluconate, nitrilotriacetic acid, tartaric acid, citric acid, and salts thereof; preferably, the salt is a sodium salt, a potassium salt, or an ammonium salt; in some embodiments, the chelating agent is selected from ethylenediaminetetraacetic acid and/or disodium ethylenediaminetetraacetic acid (i.e., disodium edetate).

According to an embodiment of the present disclosure, in the pharmaceutical composition, the chelating agent is 0.01-1 part by weight, for example, 0.05-0.5 parts, exemplarily 0.05 parts, 0.1 parts, 0.2 parts, or 0.5 parts.

According to an embodiment of the present disclosure, the pH regulator is selected from a pH buffer solution, preferably a pH buffer solution of an organic acid and/or a salt thereof; for example, one or more of a citric acid/sodium citrate buffer solution, a tartrate buffer solution, an oxalate buffer solution, and an acetic acid/acetate buffer solution, and preferably the citric acid/sodium citrate buffer solution.

According to an embodiment of the present disclosure, the solvent is selected from water, preferably purified water.

According to an embodiment of the present disclosure, in the pharmaceutical composition, the solvent is 20-60 parts by weight, for example, 25-55 parts, and exemplarily 28 parts, 28.75 parts, 30 parts, 35 parts, 40 parts, 45 parts, 47.75 parts, 48.75 parts, or 49.25 parts.

According to an embodiment of the present disclosure, the pharmaceutical composition further optionally includes a thickener; and/or optionally includes an antioxidant; and/or optionally includes a preservative.

According to an embodiment of the present disclosure, the thickener is selected from one or more of sodium carboxymethyl cellulose, hydroxypropyl methylcellulose, povidone, and polyvinyl alcohol, and preferably sodium carboxymethyl cellulose.

According to an embodiment of the present disclosure, in the pharmaceutical composition, the thickener is 0.01-1 part by weight, for example, 0.05-0.5 parts, and exemplarily 0.05 parts, 0.1 parts, 0.2 parts, or 0.5 parts.

According to an embodiment of the present disclosure, the antioxidant is selected from one or both of propyl gallate and butylated hydroxytoluene (BHT).

According to an embodiment of the present disclosure, in the pharmaceutical composition, the antioxidant is 0.01-0.2 parts by weight, for example, 0.05-0.15 parts, and exemplarily 0.05 parts, 0.1 parts, or 0.12 parts.

According to an embodiment of the present disclosure, the preservative is selected from one or more of methyl paraben, ethyl paraben, propyl paraben, and benzalkonium chloride, and preferably methyl paraben.

According to an embodiment of the present disclosure, in the pharmaceutical composition, the preservative is 0.01-0.3 parts by weight, for example, 0.05-0.15 parts, and exemplarily 0.1 parts, 0.1 parts, or 0.15 parts.

According to an embodiment of the present disclosure, the pharmaceutical composition has a pH of 4-6, and preferably 4.5-5.5, for example, 4.5, 5, 5.1, 5.2, or 5.5.

In some embodiments, the above humectant and penetration enhancer also serve as solvents for the compound represented by Formula (I) or Formula (I-1) or pharmaceutically acceptable salts thereof, the antioxidant and/or preservative.

According to an embodiment of the present disclosure, the pharmaceutical composition includes the following components in parts by weight: 0.1-8 parts of the active ingredient, 10-35 parts of the humectant, 10-50 parts of the penetration enhancer, 0.01-1 part of the chelating agent, 0.01-0.2 parts of the antioxidant, 0.01-0.3 parts of the preservative, 20-60 parts of the solvent, and the pH regulator;
the active ingredient is the compound represented by Formula (I), preferably the E-type isomer represented by Formula (I-1); and
preferably, the pharmaceutical composition has a pH of 4 to 6.

According to an embodiment of the present disclosure, the pharmaceutical composition includes the following components in parts by weight: 0.1-8 parts of the active ingredient, 10-35 parts of the humectant, 10-50 parts of the penetration enhancer, 0.01-1 part of the chelating agent, 0.01-1 part of the thickener, 0.01-0.2 parts of the antioxidant, 0.01-0.3 parts of the preservative, 20-60 parts of the solvent, and the pH regulator;
the active ingredient is the compound represented by Formula (I), preferably the E-type isomer represented by Formula (I-1); and
preferably, the pharmaceutical composition has a pH of 4 to 6.

In some embodiments, the pharmaceutical composition includes the following components in parts by weight: 0.5-5 parts of the active ingredient, 15-30 parts of the humectant, 15-45 parts of the penetration enhancer, 0.05-0.5 parts of the chelating agent, 0.05-0.15 parts of the antioxidant, 0.05-0.15 parts of the preservative, 25-55 parts of the solvent, and the pH regulator;
the active ingredient is the compound represented by Formula (I), preferably the E-type isomer represented by Formula (I-1); and
preferably, the pharmaceutical composition has a pH of 4 to 6.

In some embodiments, the pharmaceutical composition includes the following components in parts by weight: 0.5-5 parts of the active ingredient, 15-30 parts of the humectant, 15-45 parts of the penetration enhancer, 0.05-0.5 parts of the chelating agent, 0.05-0.5 parts of the thickener, 0.05-0.15 parts of the antioxidant, 0.05-0.15 parts of the preservative, 25-55 parts of the solvent, and the pH regulator;
the active ingredient is the compound represented by Formula (I), preferably the E-type isomer represented by Formula (I-1);
preferably, the pharmaceutical composition has a pH of 4 to 6.

In some embodiments, the pharmaceutical composition includes the following components in parts by weight: 0.1-8 parts of the E-type isomer represented by Formula (I-1), 10-35 parts of propylene glycol, 10-50 parts of diethylene glycol monoethyl ether, 0.01-1 part of disodium edetate, 0.01-0.2 parts of propyl gallate, 0.01-0.3 parts of methyl paraben, 20-60 parts of water, and a pH regulator; preferably, the pharmaceutical composition has a pH of 4 to 6.

In some embodiments, the pharmaceutical composition includes the following components in parts by weight: 0.1-8 parts of the E-type isomer represented by Formula (I-1), 10-35 parts of propylene glycol, 10-50 parts of diethylene glycol monoethyl ether, 0.01-1 part of disodium edetate, 0.01-1 part of sodium carboxymethyl cellulose, 0.01-0.2 parts of propyl gallate, 0.01-0.3 parts of methyl paraben, 20-60 parts of water, and a pH regulator; preferably, the pharmaceutical composition has a pH of 4 to 6.

In some embodiments, the pharmaceutical composition includes the following components in parts by weight: 0.5-5 parts of the E-type isomer represented by Formula (I-1), 15-30 parts of propylene glycol, 15-45 parts of diethylene glycol monoethyl ether, 0.05-0.15 parts of disodium edetate, 0.05-0.15 parts of propyl gallate, 0.05-0.15 parts of methyl paraben, 25-55 parts of water. and a pH regulator: preferably, the pharmaceutical composition has a pH of 4 to 6.

In some embodiments, the pharmaceutical composition includes the following components in parts by weight: 0.5-5 parts of the E-type isomer represented by Formula (I-1), 15-30 parts of propylene glycol, 15-45 parts of diethylene glycol monoethyl ether, 0.05-0.15 parts of disodium edetate, 0.05-0.15 parts of sodium carboxymethyl cellulose, 0.05-0.15 parts of propyl gallate, 0.05-0.15 parts of methyl paraben, 25-55 parts of water, and a pH regulator; preferably, the pharmaceutical composition has a pH of 4 to 6.

According to an exemplary embodiment of the present disclosure, the pharmaceutical composition includes the following components in parts by weight: 1 part of the E-type isomer represented by Formula (I-1), 30 parts of propylene glycol, 25 parts of diethylene glycol monoethyl ether, 0.05 parts of disodium edetate, 0.05 parts of propyl gallate, 0.1 parts of methyl paraben, 43.75 parts of water, and a pH regulator, preferably the pH regulator is a citric acid/sodium citrate buffer solution; and preferably, the pharmaceutical composition has a pH of 4 to 6.

According to an exemplary embodiment of the present disclosure, the pharmaceutical composition includes the following components in parts by weight: 1 part of the E-type isomer represented by Formula (I-1), 30 parts of propylene glycol, 25 parts of diethylene glycol monoethyl ether, 0.05 parts of disodium edetate, 0.05 parts of sodium carboxymethyl cellulose, 0.05 parts of propyl gallate, 0.1 parts of methyl paraben, 43.75 parts of water, and a pH regulator, preferably the pH regulator is a citric acid/sodium citrate buffer solution; and preferably, the pharmaceutical composition has a pH of 4 to 6.

According to an exemplary embodiment of the present disclosure, the pharmaceutical composition includes the following components in parts by weight: 0.5 parts of the E-type isomer represented by Formula (I-1), 30 parts of propylene glycol, 41 parts of diethylene glycol monoethyl ether, 0.05 parts of disodium edetate, 0.05 parts of propyl gallate, 0.1 parts of methyl paraben, 28.25 parts of water, and a pH regulator, preferably the pH regulator is a citric acid/sodium citrate buffer solution; and preferably, the pharmaceutical composition has a pH of 4 to 6.

According to an exemplary embodiment of the present disclosure, the pharmaceutical composition includes the following components in parts by weight: 0.5 parts of the E-type isomer represented by Formula (I-1), 30 parts of propylene glycol, 41 parts of diethylene glycol monoethyl ether, 0.05 parts of disodium edetate, 0.05 parts of sodium carboxymethyl cellulose, 0.05 parts of propyl gallate, 0.1 parts of methyl paraben, 28.25 parts of water, and a pH regulator, preferably the pH regulator is a citric acid/sodium citrate buffer solution; and preferably, the pharmaceutical composition has a pH of 4 to 6.

According to an exemplary embodiment of the present disclosure, the pharmaceutical composition includes the following components in parts by weight: 0.5 parts of the E-type isomer represented by Formula (I-1), 25 parts of propylene glycol, 25 parts of diethylene glycol monoethyl ether, 0.05 part of disodium edetate, 0.05 parts of propyl gallate, 0.1 parts of methyl paraben, 49.25 parts of water, and a pH regulator, preferably the pH regulator is a citric acid/sodium citrate buffer solution; and preferably, the pharmaceutical composition has a pH of 4 to 6.

According to an exemplary embodiment of the present disclosure, the pharmaceutical composition includes the following components in parts by weight: 0.5 parts of the E-type isomer represented by Formula (I-1), 25 parts of propylene glycol, 25 parts of diethylene glycol monoethyl ether, 0.05 parts of disodium edetate, 0.05 parts of sodium carboxymethyl cellulose, 0.05 parts of propyl gallate, 0.1 parts of methyl paraben, 49.25 parts of water, and a pH regulator, preferably the pH regulator is a citric acid/sodium citrate buffer solution; and preferably, the pharmaceutical composition has a pH of 4 to 6.

According to an exemplary embodiment of the present disclosure, the pharmaceutical composition includes the following components in parts by weight: 1 part of the E-type isomer represented by Formula (I-1), 25 parts of propylene glycol, 25 parts of diethylene glycol monoethyl ether, 0.05 parts of disodium edetate, 0.05 parts of propyl gallate, 0.1 parts of methyl paraben, 48.75 parts of water, and a pH regulator, preferably the pH regulator is a citric acid/sodium citrate buffer solution; and preferably, the pharmaceutical composition has a pH of 4 to 6.

According to an exemplary embodiment of the present disclosure, the pharmaceutical composition includes the following components in parts by weight: 1 part of the E-type isomer represented by Formula (I-1), 25 parts of propylene glycol, 25 parts of diethylene glycol monoethyl ether, 0.05 parts of disodium edetate, 0.05 parts of sodium carboxymethyl cellulose, 0.05 parts of propyl gallate, 0.1 parts of methyl paraben, 48.75 parts of water, and a pH regulator, preferably the pH regulator is a citric acid/sodium citrate buffer solution; and preferably, the pharmaceutical composition has a pH of 4 to 6.

According to an exemplary embodiment of the present disclosure, the pharmaceutical composition includes the following components in parts by weight: 2 parts of the E-type isomer represented by Formula (I-1), 25 parts of propylene glycol, 25 parts of diethylene glycol monoethyl ether, 0.05 parts of disodium edetate, 0.05 parts of propyl gallate, 0.1 parts of methyl paraben, 47.75 parts of water, and a pH regulator, preferably the pH regulator is a citric acid/sodium citrate buffer solution; and preferably, the pharmaceutical composition has a pH of 4 to 6.

According to an exemplary embodiment of the present disclosure, the pharmaceutical composition includes the following components in parts by weight: 2 parts of the E-type isomer represented by Formula (I-1), 25 parts of propylene glycol, 25 parts of diethylene glycol monoethyl ether, 0.05 parts of disodium edetate, 0.05 parts of sodium carboxymethyl cellulose, 0.05 parts of propyl gallate, 0.1 parts of methyl paraben, 47.75 parts of water, and a pH regulator, preferably the pH regulator is a citric acid/sodium citrate buffer solution; and preferably, the pharmaceutical composition has a pH of 4 to 6.

Preferably, a sum of total parts by weight of the above components is 100 parts.

According to an embodiment of the present disclosure, the pharmaceutical composition may further include a second active ingredient.

The present disclosure further provides a preparation method of the above pharmaceutical composition, including mixing the components to obtain the same.

Preferably, the preparation method includes the following steps:
(1) mixing the active ingredient with the humectant and the penetration enhancer to form a solution containing the active ingredient;
   or mixing the active ingredient, antioxidant, and preservative with the humectant and the penetration enhancer to form a solution containing the active ingredient;
(2) mixing the chelating agent and the thickener with the solvent to form a mixed solution; preferably, first dissolving the chelating agent in the solvent, and then adding the thickener for dispersion and dissolution;
(3) adding the mixed solution into the solution containing the active ingredient, mixing uniformly, and then adding the pH regulator to adjust a pH of the system, preferably to 4-6;
(5) adding the solvent to make up weight to obtain the pharmaceutical composition.

The present disclosure further provides use of the above pharmaceutical composition in the preparation of a pharmaceutical formulation; preferably the pharmaceutical formulation is a solution-type formulation, for example, a spray or an aerosol.

The present disclosure further provides a pharmaceutical formulation, preferably a solution-type formulation, more preferably a spray or aerosol, including the above pharmaceutical composition.

According to an embodiment of the present disclosure, in the pharmaceutical formulation, the active ingredient (i.e., at least one of the compound represented by Formula (I) and pharmaceutically acceptable salts thereof) has a mass percentage of 0.1-8%, for example, 0.5-5%, and exemplarily 0.5%, 0.75%, 1%, 1.5%, 2%, 3%, or 4%.

According to an embodiment of the present disclosure, the pharmaceutical composition or the pharmaceutical formulation is used for treating an autoimmune inflammatory disease, preferably psoriasis, atopic dermatitis, and/or seborrheic dermatitis; and
preferably, the pharmaceutical composition or the pharmaceutical formulation is used for treating seborrheic dermatitis or the above diseases occurring at hair-bearing sites, and is especially suitable for seborrheic dermatitis or psoriasis occurring at hair-bearing sites, for example, the hair-bearing sites include, but are not limited to, the scalp and axillae; preferably scalp psoriasis or seborrheic dermatitis of the scalp.

The present disclosure provides use of the above pharmaceutical composition in preparation of a medicament for the treatment and/or prevention of an autoimmune inflammatory disease; wherein the autoimmune disease is preferably selected from psoriasis, atopic dermatitis, and/or seborrheic dermatitis, more preferably seborrheic dermatitis or psoriasis occurring at hair-bearing sites, and most preferably seborrheic dermatitis of the scalp or scalp psoriasis.

According to an embodiment of the present disclosure, the pharmaceutical formulation includes a container for containing the pharmaceutical composition. Preferably, the container is a light-resistant, sealed container.

**In** an embodiment, the pharmaceutical formulation further includes a spray or aerosol device for spraying the pharmaceutical composition onto a diseased site in the form of a spray or aerosol, preferably the diseased site is a hair-bearing diseased site, for example, which includes but is not limited to the scalp and axillae.

**In** an embodiment. the pharmaceutical formulation further includes a pronellant.

**In** one embodiment, a route of administration of the pharmaceutical composition or the pharmaceutical formulation is topical administration, or further in combination with an oral formulation or another topical formulation.

The present disclosure further provides a method for treating an autoimmune inflammatory disease, preferably psoriasis, atopic dermatitis, and seborrheic dermatitis, especially the above diseases occurring at hair-bearing sites, including administering to a patient an effective amount or a therapeutically effective amount of the pharmaceutical composition or the pharmaceutical formulation.

The present disclosure further provides use of the above pharmaceutical composition in preparation of a medicament for the treatment and/or prevention of a disease caused by *Pityrosporum ovale;* further, the disease is seborrheic dermatitis caused by *Pityrosporum ovale.*

The present disclosure further provides a method for treating and/or preventing a disease caused by *Pityrosporum ovale,* including administering to a patient an effective amount or a therapeutically effective amount of the pharmaceutical composition or the pharmaceutical formulation.

### Definitions and explanations of terms

Unless otherwise specified, the term definitions contained in the specifications and claims of the present application, including definition as an example, exemplary definition, preferred definitions, definitions recorded in tables, and definitions of specific compounds in embodiments, and may be combined and integrated with each other arbitrarily. Such combinations and integrations should fall within the recording scope of the specifications in the present application.

The term "effective amount" or "therapeutically effective amount" refers to the amount of the compound in the present disclosure that is sufficient to achieve the intended application (including but not limited to the treatment of the diseases defined below). The therapeutically effective amount may vary depending on factors such as the intended application *(in vitro* or *in vivo),* the subject being treated, and the characteristics of the disease, such as the subject's weight and age, the severity of the disease, and the route of administration, which can be readily determined by a person skilled in the art. The specific dosage will vary depending on factors such as the selected specific compound, the administration plan upon which it is based, whether it is administered in combination with other compounds, the timing of administration, the tissues to which it is administered, and the physical delivery system employed.

The term "optionally" or "optional" means that the event or circumstance subsequently described may, but does not necessarily, occur, and includes both cases where the event or circumstance occurs and where it does not occur. For example, "optionally including an antioxidant" includes both the case of including an antioxidant and the case of not including an antioxidant.

The term "patient" refers to individuals who need preventive measures or treatment for diseases related to anaerobic bacteria, wherein the patient is a mammal, such as a rodent, cattle, a pig, a dog, a cat, and a primate, especially a human.

### Beneficial effects

The present disclosure provides a pharmaceutical composition for preparing a spray or an aerosol, which has good physical and chemical stability. The composition/spray is used for treating autoimmune inflammatory diseases, preferably psoriasis, atopic dermatitis, and seborrheic dermatitis, and is especially suitable for treating seborrheic dermatitis and the above diseases occurring at hair-bearing sites (such as scalp psoriasis). It has a good therapeutic effect on seborrheic dermatitis, and improves the problems in scalp psoriasis such as difficulty in administration at hair-bearing sites, poor therapeutic effect, and pain and infection caused by application of conventional topical external drugs, thereby significantly improving patient compliance and quality of life, improving clinical efficacy and safety, and being conducive to the long-term management of autoimmune inflammatory diseases.

### BRIEF DESCRIPTION OF DRAWINGS

FIG. 1 shows an experimental result of intradermal retention amounts of Examples 1-5.
FIG. 2 shows an experimental result of intradermal retention amounts of Example 4 and the control group.
FIG. 3 shows an experimental result of cumulative permeation amounts of Example 4 and the control group.
FIG. 4 shows an experimental result of average cumulative permeation amounts of Example 4, Example 12, and the control group;
FIG. 5 shows an experimental result of skin retention amounts of Example 4, Example 12, and the control group.
FIG. 6 shows a clinical effect of changes in erythema index in treatment of seborrheic dermatitis by Example 12.
FIG. 7 shows a clinical effect of changes in desquamation index in the treatment of seborrheic dermatitis by Example 12.

### DETAILED DESCRIPTION

The technical solutions of the present disclosure will be further described hereinafter through the specific examples. It is to be understood that the examples described below are intended to illustrate and explain the present disclosure but are not construed to limit the protective scope thereof. Any technology realized based on the aforementioned content of the present disclosure should fall within the scope intended to be protected by the present disclosure.

Unless otherwise stated, the raw materials and reagents used in the following examples are commercial products or can be prepared by known methods.

### Examples 1-5

The formulations of Examples 1-5 are shown in Table 1, and a preparation method for the pharmaceutical compositions is as follows:
1) the E-type isomer of the compound represented by Formula (I), methyl paraben, and propyl gallate were dissolved in a mixed solvent of propylene glycol and diethylene glycol monoethyl ether;
2) disodium edetate was dissolved in purified water, and then sodium carboxymethyl cellulose was dispersed and dissolved to obtain a mixed solution;
3) a buffer solution of citric acid and sodium citrate was prepared;
4) the mixed solution from step 2) was added to a drug-containing solution from step 1), stirred and mixed uniformly, and a pH of the system was adjusted to 4-6 with the buffer solution from step 3); and
5) purified water was added to make up to a total weight to obtain the pharmaceutical composition.

**Table 1**

| **Component** | **Example 1** | **Example 2** | **Example 3** | **Example 4** | **Example 5** |
|---|---|---|---|---|---|
| | **Percentage % (w/w)** | **Percentage % (w/w)** | **Percentage % (w/w)** | **Percentage % (w/w)** | **Percentage % (w/w)** |
| E-type isomer of the compound represented by Formula (I) | 1 | 0.50 | 0.50 | 1.00 | 2.00 |
| Propylene glycol | 30.00 | 30.00 | 25.00 | 25.00 | 25.00 |
| Diethylene glycol monoethyl ether | 25.00 | 41.00 | 25.00 | 25.00 | 25.00 |
| Propyl gallate | 0.05 | 0.05 | 0.05 | 0.05 | 0.05 |
| Methyl paraben | 0.10 | 0.10 | 0.10 | 0.10 | 0.10 |
| Disodium edetate | 0.05 | 0.05 | 0.05 | 0.05 | 0.05 |
| Sodium carboxymethy 1 cellulose | 0.05 | 0.05 | 0.05 | 0.05 | 0.05 |
| Citric acid | Appropriate amount | Appropriate amount | Appropriate amount | Appropriate amount | Appropriate amount |
| Sodium citrate | Appropriate amount | Appropriate amount | Appropriate amount | Appropriate amount | Appropriate amount |
| Purified water | About 43.75 | About 28.25 | About 49.25 | About 48.75 | About 47.75 |
| Total amount | 100 | 100 | 100 | 100 | 100 |

### Example 6

The pharmaceutical compositions of Examples 1-5 were prepared into sprays. The active ingredient in the spray was sprayed out in the form of fine mist droplets and was uniformly distributed.

### Example 7

The pharmaceutical compositions of Examples 1-5 were prepared into aerosols. The active ingredient in the aerosol was sprayed out in the form of fine mist droplets and was uniformly distributed.

### Example 8 In vitro transdermal test of Examples 1-5

The intradermal retention amounts of Examples 1-5 were tested according to the following method.

### Experimental method:

Sample from Example 4 and a control group (commercially available Benvitimod cream: produced by Guangdong Zhonghao Pharmaceutical Co., Ltd; trade name: Symbiox^{®}, and the content of Benvitimod: 1%) were subjected to *in vitro* transdermal tests to evaluate intradermal retention amount and cumulative permeability. Pig skin (with a thickness of 0.8-1.0 mm) was mounted on the LOGAN System 918 vertical diffusion cell, exposing a surface area of 1.77 cm². The diffusion cell was connected to a multi-channel pump with a rotational speed of 600 rpm and an accepting liquid of physiological saline (0.9% sodium chloride solution). Each cell was placed in a heating manifold to equilibrate the temperature, ensuring a skin surface temperature of 32°C (at least 30 min before administration). The sample loading amount was 0.3 g. The accepting liquid was collected at 4, 8, 12, 16, 24 h to measure the active ingredient that penetrated the skin. At the end of the 24 h sampling, the skin was wiped with a cotton swab to remove any residual samples considered impermeable to the skin, and the skin layer was cut into pieces and methanol was added for ultrasonic extraction of the drug. The concentration of the recovered drug was used to calculate the retention amount in the skin.

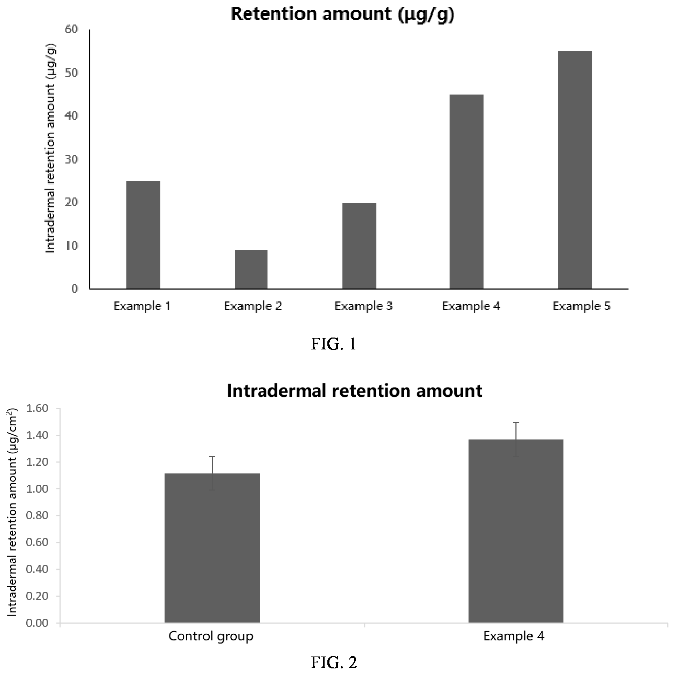

The test results are shown in FIG. 1 , showing that the intradermal retention amounts of Example 1, Example 2, and Example 3 were all significantly lower than those of Example 4 and Example 5, and Example 4 was determined as the optimized formulation for subsequent screening.

### Example 9 Optimization of antioxidant

The antioxidant propyl gallate in Example 4 was changed to butylated hydroxytoluene, and the stability of the new formulation was further observed. The specific formulation is shown in Table 2 below:

**Table 2**

| **Component** | **Example 9** |
|---|---|
| | **Percentage % (w/w)** |
| E-type isomer of the compound represented by Formula (I) | 1.00 |
| Propylene glycol | 25.00 |
| Diethylene glycol monoethyl ether | 25.00 |
| Butylated hydroxytoluene | 0.05 |
| Methyl paraben | 0.10 |
| Disodium edetate | 0.05 |
| Sodium carboxymethyl cellulose | 0.05 |
| Citric acid | Appropriate amount |
| Sodium citrate | Appropriate amount |
| Purified water | About 48.75 |
| Total amount | 100 |

### a) Stability comparison:

Equal amounts of the composition of Example 4 and the composition of Example 9 were weighed into light-shielding sealed vials for liquid chromatography, and a total of 3 groups were set and placed at room temperature and at 60°C, respectively, and the physical and chemical stability of samples at 0 day, 5 days at 60°C, and 10 days at 60°C was observed and measured by liquid chromatography. The related substance determination method is shown in Table 3-2, and the specific results are shown in Table 3-1. The results showed that Example 9 was examined of a newly detected impurity with RRT 2.19 after 5 days and 10 days at high temperature, while Example 4 did not show any new impurity. Example 4 had superior stability compared with Example 9.

**Table 3-1**

| **Batch** | **Saving condition** | **pH val ue** | **Solution clarity and color** | **Related substances (%)** | | |
|---|---|---|---|---|---|---|
| | | | | **RRT 1.22** | **RRT 2.19** | **Total impuriti es** |
| | 0 day | 5.1 | Lighter than Chinese Pharmacopoeia Orange Yellow No. 0.5 | 0.04 | ND | 0.04 |
| Example 4 | High temperature (60°C) for 5 days | 5.1 | Darker than Chinese Pharmacopoeia Orange Yellow No. 1, and lighter than Chinese Pharmacopoeia Orange Yellow No. 2 | 0.03 | ND | 0.03 |
| | High temperature (60°C) for 10 days | 5.1 | Darker than Chinese Pharmacopoeia Orange Yellow No. 5, and lighter than Chinese Pharmacopoeia Orange Yellow No. 6 | 0.04 | ND | 0.04 |
| Example 9 | 0 day | 5.2 | Lighter than Chinese Pharmacopoeia Orange Yellow No. 0.5 | 0.04 | ND | 0.04 |
| | High temperature (60°C) for 5 days | 5.2 | Darker than Chinese Pharmacopoeia Orange Yellow No. 2, and lighter than Chinese Pharmacopoeia Orange Yellow No. 3 | 0.03 | 0.01 | 0.03 |
| | High temperature (60°C) for 10 days | 5.2 | Darker than Chinese Pharmacopoeia Orange Yellow No. 6, and lighter than Chinese Pharmacopoeia Orange Yellow No. 7 | 0.04 | 0.02 | 0.06 |

Related substance determination method: octadecylsilane-bonded silica gel was used as a filler; water was used as a mobile phase A, acetonitrile was used as a mobile phase B, and gradient elution was performed according to Table 3-2; the flow rate was 1.0 mL per minute; the column temperature was 30°C; the detection wavelength was 220 nm; and the injection volume was 25 µL.

**Table 3-2**

| Time (min) | Mobile phase A (%) | Mobile phase B (%) |
|---|---|---|
| 0 | 70 | 30 |
| 15 | 70 | 30 |
| 40 | 20 | 80 |
| 55 | 20 | 80 |
| 56 | 70 | 30 |
| 65 | 70 | 30 |

### Example 10 Stability test

Equal amounts of the composition of Example 4 were weighed into light-shielding sealed vials for liquid chromatography, and a total of 4 groups were set and placed at room temperature, 60°C, and 40°C, respectively, and the physical and chemical stability of samples at 0 day, 10 days at 60°C, 30 days at 60°C, and 3 months at 40°C was observed and measured by liquid chromatography. The specific results are shown in Table 4. The results showed that the composition of Example 4 had excellent stability. The related substance test method was as described in Example 9.

**Table 4**

| Item | Inspection condition | Solution color | pH | Largest single impurity | Total impurities |
|---|---|---|---|---|---|
| Example 4 | 0 day | Orange yellow solution | 5.2 | 0.03% | 0.03% |
| | 60°C for 10 days | Orange yellow solution | 5.2 | 0.04% | 0.04% |
| | 60°C for 30 days | Orange yellow solution | 5.2 | 0.04% | 0.06% |
| | 40°C for 3 months | Orange yellow solution | 5.2 | 0.10% | 0.33% |

### Example 11 Intradermal retention amount and cumulative permeation amount test

The intradermal retention amount and cumulative permeation amount of Example 4 and the control group (commercially available Benvitimod cream: produced by Guangdong Zhonghao Pharmaceutical Co., Ltd; trade name: Symbiox^{®}, and the content of Benvitimod: 1%) were tested according to the following method.

### Experimental method:

Sample from Example 4 and the control group were subjected to *in vitro* transdermal tests to evaluate intradermal retention amount and cumulative permeability. Pig skin (with a thickness of 0.8-1.0 mm) was mounted on the LOGAN System 918 vertical diffusion cell, exposing a surface area of 1.77 cm². The diffusion cell was connected to a multi-channel pump with a rotational speed of 600 rpm and an accepting liquid of PBS (with a pH of 7.4). Each cell was placed in a heating manifold to equilibrate the temperature, ensuring a skin surface temperature of 32°C (at least 30 min before administration). Samples were loaded at a dose of 10 mg test sample per square centimeter. The accepting liquid was collected at 2, 4, 8, 20, 24 h to measure the active ingredient that penetrated the skin. At the end of the 24 h sampling, the skin was wiped with a cotton swab to remove any residual samples considered impermeable to the skin, and the skin layer was cut into pieces and methanol was added for ultrasonic extraction of the drug. The concentration of the recovered drug was used to calculate the retention amount in the skin.

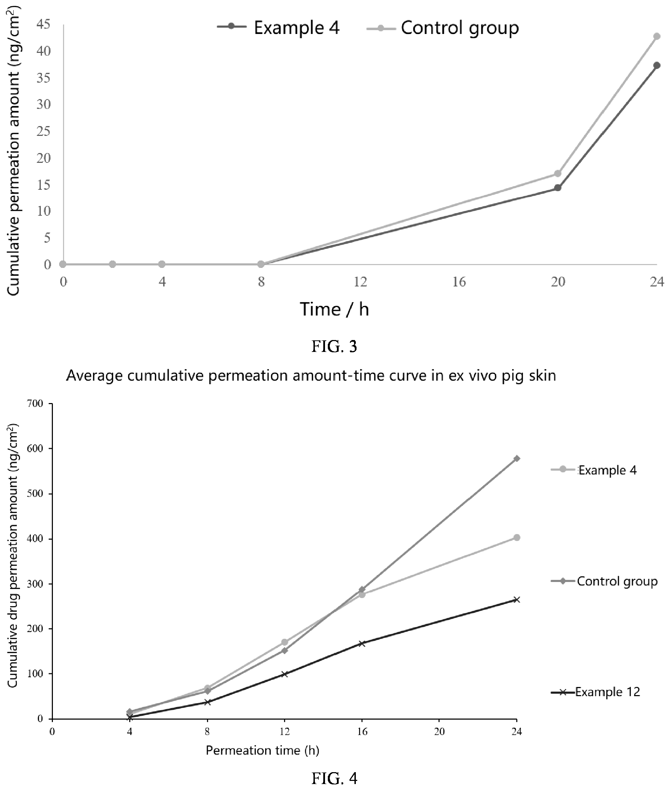

The test results are shown in FIGS. 2 and 3, showing that both the intradermal retention amount and the cumulative permeation amount of Example 4 were superior to those of the control group.

### Example 12 Optimization of thickener

The applicant considered that the use of a thickener in the formulation might increase the viscosity of the drug solution and at the same time might affect the *in vitro* transdermal effect of the formulation, thereby affecting patient compliance in using the formulation. The optimized formulation is shown in Table 5:

**Table 5**

| **Component** | **Example 12** |
|---|---|
| | **Percentage % (w/w)** |
| E-type isomer of the compound represented by Formula (I) | 1.00 |
| Propylene glycol | 25.00 |
| Diethylene glycol monoethyl ether | 25.00 |
| Propyl gallate | 0.05 |
| Methyl paraben | 0.10 |
| Disodium edetate | 0.05 |
| Citric acid | Appropriate amount |
| Sodium citrate | Appropriate amount |
| Purified water | About 48.80 |
| Total amount | 100 |

The formulation containing sodium carboxymethyl cellulose, i.e., Example 4, had significantly increased viscosity and poor patient compliance; the formulation without sodium carboxymethyl cellulose, i.e., Example 12, could be uniformly dispersed on the skin, had low fluidity, and had good patient compliance.

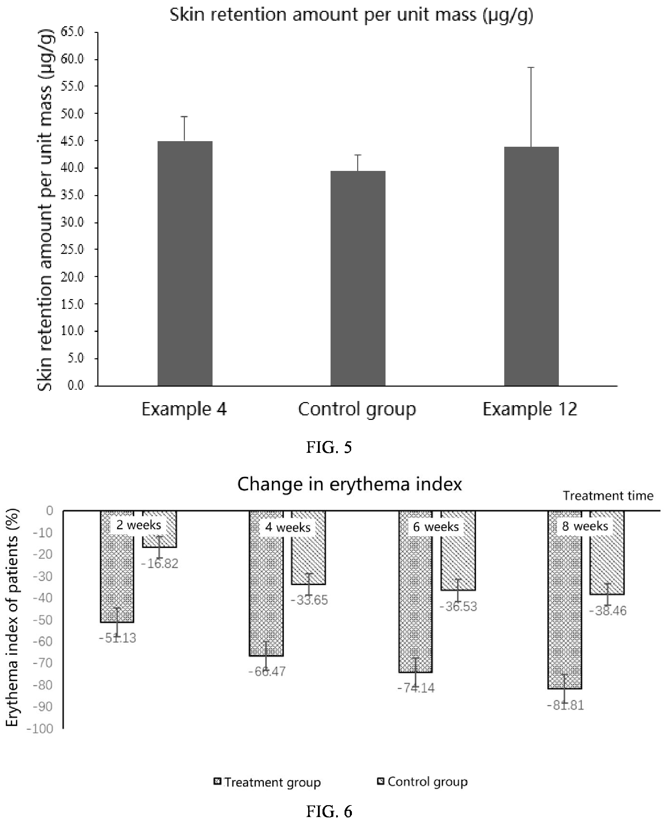

Using the *in vitro* transdermal experimental procedure in Example 11, the *in vitro* transdermal conditions of Example 4 and Example 12 (including average cumulative permeation amount and skin retention amount per unit mass) were observed. The test results are shown in FIGS. 4 and 5, respectively. It can be seen that the retention amounts in the *in vitro* transdermal experiments of Example 4 and Example 12 were close, and both were slightly higher than that of the control group (commercially available Benvitimod cream: produced by Guangdong Zhonghao Pharmaceutical Co., Ltd; trade name: Symbiox^{®}, and the content of Benvitimod: 1%), while the average cumulative permeation amount of Example 12 (sample without sodium carboxymethyl cellulose) was lower than that of Example 4 and the control group, and was only half of that of the control group. Therefore, the safety risk of the sample of Example 12 was reduced.

### Example 13 Stability test

Equal amounts of the composition of Example 12 were weighed into light-shielding sealed vials and subjected to an accelerated test investigation (40°C ± 2°C / 75% ± 5% RH). The content determination method is shown in Table 6-2, and the specific results are shown in Table 6-1. The results showed that after 6 months of accelerated test, the composition of Example 12 met the quality standard and had excellent stability.

**Table 6-1**

| Item | Inspection condition | Solution color | pH | Largest single impurity | Total impurities | Content |
|---|---|---|---|---|---|---|
| | 0 day | Colorless clear solution | 5.2 | <0.05% | <0.05% | 101.0 |
| | 1 month | Colorless clear solution | 5.2 | <0.05% | <0.05% | 101.2 |
| Example 12 | 2 months | Light orange yellow solution | 5.1 | <0.05% | <0.05% | 99.4 |
| | 3 months | Light orange yellow solution | 5.1 | 0.06% | 0.06% | 100.3 |
| | 6 months | Light orange yellow solution | 5.1 | 0.07% | 0.07% | 100.1 |

### The related substance determination method was as described in Example 9.

Content determination method: octadecylsilane-bonded silica gel was used as a filler; water was used as a mobile phase A, acetonitrile was used as a mobile phase B, and gradient elution was performed according to Table 6-2; the flow rate was 1.0 mL per minute; the column temperature was 30°C; the detection wavelength was 220 nm; and the injection volume was 25 µL.

**Table 6-2**

| Time (min) | Mobile phase A (%) | Mobile phase B (%) |
|---|---|---|
| 0 | 50 | 50 |
| 15 | 20 | 80 |
| 20 | 20 | 80 |
| 21 | 50 | 50 |
| 30 | 50 | 50 |

### Example 14 Pharmacokinetic test

After transdermal administration to Bama miniature pigs of the composition of Example 12 with a Benvitimod content of 1% and the control group (commercially available Benvitimod cream: produced by Guangdong Zhonghao Pharmaceutical Co., Ltd; trade name: Symbiox^{®}, and the content of Benvitimod: 1%), the skin pharmacokinetic characteristics of the common active ingredient Benvitimod in both were investigated, and the differences between the two were compared.

Six Bama miniature pigs were selected, with half male and half female. After transdermal application of 10 µL/cm² of the composition of Example 12 and 10 mg/cm² of the control group to the left and right sides of the dorsal skin of the pigs, respectively, for twice (dosing interval of 10 h), skin from the administration area was excised at 1, 2, 4, 6, 8, 12, 24, 36, 48, and 72 h after the last administration (according to the design, dosing was performed at different time points, and finally each group reached the endpoint simultaneously before the administered skin was excised). After wiping the surface ointment clean with alcohol, the stratum corneum was repeatedly stripped off with adhesive tape. In addition, skin from a blank area was taken as the 0 h point, and the stratum corneum was repeatedly stripped off with adhesive tape and stored at -20°C. The concentration of Benvitimod in the skin was determined by HPLC-MS/MS, and PK parameters were calculated using the non-compartmental model of DAS 3.3. Statistical analysis was performed using SPSS.

The results are shown in Table 7. After local administration of the composition of Example 12 with a Benvitimod content of 1% and the control group to Bama miniature pigs, respectively, the exposure amount of Benvitimod in the skin in the Example 12 composition group was about 3.26 times that of the control group, and the AUC₍₀₋ₜ₎ values were 669 ± 187 h*µg/g and 205 ± 39.2 h*µg/g, respectively.

**Table 7 Pharmacokinetic parameters in skin after transdermal administration of Example 12/control group to Bama miniature pigs**

| Parameter | Unit | Example 12 | Control group |
|---|---|---|---|
| AUC₍₀₋ₜ₎ | h*µg/g | 669±187 | 205±39.2 |
| Cₘₐₓ | µg/g | 24.2±10.9 | 6.40±1.45 |
| Tₘₐₓ | h | 18(2,48) | 5(1,48) |

| | | | |
|---|---|---|---|
| Note: Tₘₐₓ is the median (minimum, maximum). | | | |

### Example 15 Sensitization test

A Buehler test was performed in adult guinea pigs to evaluate the potential local sensitization of the pharmaceutical compositions of Example 4 and Example 5.

### Experimental method:

60 healthy adult guinea pigs, half male and half female, were randomly divided by sex and body weight using the software TOXSTAT2006 into a negative control group, a positive control group, a low-dose group (Example 4, 1%), and a high-dose group (Example 5, 2%). The negative control group and the positive control group each had 10 animals (half male and half female), and the low-dose group and the high-dose group each had 20 animals (half male and half female). Before administration, the animals were clipped with electric clippers to remove hair from the skin on both sides of the dorsal spine, with an area of 3 × 3 cm² on each side.

Sensitization: on the 0th, 7th and 14th days, respectively, 0.2 mL/individual of test object was given to the left side of the hair removal area, a filter paper was pasted on the groove of the blank plaster, and the test object was contained in the filter paper and applied to the administration site, and then sealed and fixed with non-irritating medical tape. After about 6 h, the blank plaster or filter paper was removed. The animals in the negative control group were given 0.9% sodium chloride injection, the animals in the positive control group were given 2% 2,4-dinitrochlorobenzene (ultrasonically prepared with 80% ethanol), and the animals in the low-dose group (Example 4) and high-dose group (Example 5) were given the corresponding concentrations of the Example compositions (1% and 2%).

Provocation: on the 14th day after the last sensitization, the test subjects were given 0.2 mL/individual on the right side of the guinea pig hair removal area in each group 0.2% 2,4-dinitrochlorobenzene (prepared using acetone solution), About 6 h later, the test subject was removed. Allergic reaction symptoms at 1 h and 24 h after sensitization, and allergic reaction symptoms at 24 h and 48 h after provocation were observed and recorded.

The results showed that under the conditions of this test, after repeated administration of the compositions at different concentrations (1%, 2%) to adult guinea pigs, the Buehler test evaluation result was negative, indicating that with an increase in concentration, there was still no sensitization effect on the skin.

### Example 16 Clinical efficacy for seborrheic dermatitis

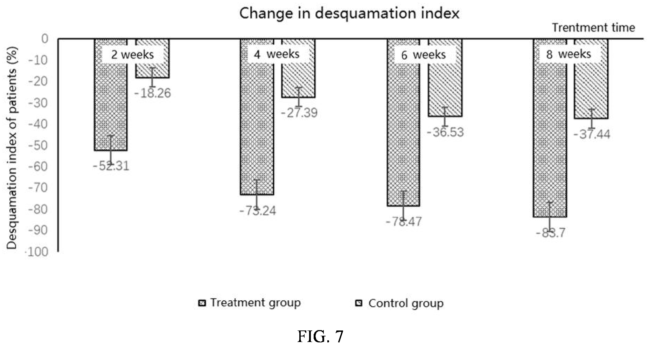

In a prospective, randomized, 8-week clinical trial, the spray composition prepared in Example 12 (treatment group) was applied to the skin of subjects with seborrheic dermatitis and administered to the affected area twice daily. The control group was administered to the affected area twice daily with the matrix components of Example 12 without Benvitimod. Changes in erythema index and desquamation index in seborrheic dermatitis were significantly improved in the second week and continued into the eighth week. By the eighth week, in the treatment group, the change of erythema index decreased by 81.81% and the change of desquamation index decreased by 83.70%. In the control group, the change of erythema index decreased by 38.46%, and the change of desquamation index decreased by 37.44%. Table 8 and FIG. 6 and FIG. 7 show the clinical efficacy of the AhR agonist Benvitimod in the treatment of patients with seborrheic dermatitis.

**Table 8 Clinical efficacy of spray composition of Example 12 in the treatment of seborrheic dermatitis**

| **Change in erythema index (%)** | | |
|---|---|---|
| Treatment period (weeks) | Treatment group | Control group |
| 2 | -51.13 | -16.82 |
| 4 | -66.47 | -33.65 |
| 6 | -74.14 | -36.53 |
| 8 | -81.81 | -38.46 |

| **Change in desquamation index (%)** | | |
|---|---|---|
| Treatment period (weeks) | Treatment group | Control group |
| 2 | -52.31 | -18.26 |
| 4 | -73.24 | -27.39 |
| 6 | -78.47 | -36.53 |
| 8 | -83.70 | -37.44 |

Based on the above experimental results, it can be seen that the pharmaceutical composition and the formulation of the present disclosure have good stability, effectiveness, and safety in the treatment of autoimmune inflammatory diseases, preferably diseases such as psoriasis, atopic dermatitis, and seborrheic dermatitis. In particular, the spray and the aerosol not only improve the stability of the active ingredient during use and prolong the service life after opening, but also reduce the treatment cost to patients and potential chemical stability risks. In addition, the drug in the formulation of the present disclosure is sprayed out in the form of fine mist droplets and is uniformly distributed, which facilitates diffusion, while reducing the irritation of friction to the wound surface during the smearing process, and reducing pain and infection from local application, and is especially suitable for treatment of disease occurring at hair-bearing sites. These formulations improve patient compliance, improve clinical efficacy and safety, enhance stability during drug use, and are conducive to the long-term management of autoimmune inflammatory diseases, preferably psoriasis, seborrheic dermatitis, atopic dermatitis, and the like.

The embodiments of the present disclosure have been described above. However, the present disclosure is not limited to the above embodiments. Any modifications, equivalent substitutions, improvements, and the like made within the spirit and principles of the present disclosure shall be included within the protection scope of the present disclosure.

## Claims

1. A pharmaceutical composition, comprising the following components: an active ingredient, a humectant, a penetration enhancer, a chelating agent, a pH regulator, and a solvent, wherein the active ingredient is selected from at least one of the compound represented by Formula (I) and pharmaceutically acceptable salts thereof:

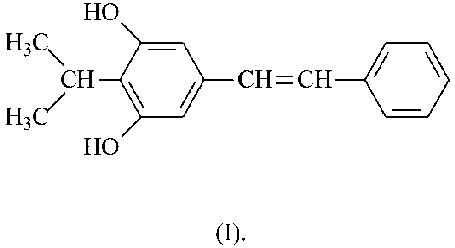

2. The pharmaceutical composition according to claim 1, wherein the pharmaceutical composition is a solution, preferably a solution-type pharmaceutical composition for spray or aerosol administration.

3. The pharmaceutical composition according to claim 1 or 2, wherein the compound represented by Formula (I) is an E-type or Z-type isomer, preferably the E-type isomer represented by Formula (I-1) below: 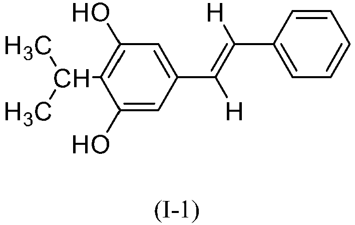
preferably, the pharmaceutically acceptable salt is selected from an addition salt of the compound of Formula (I) with an alkali;
preferably, in the pharmaceutical composition, only the compound represented by Formula (I) is used as the active ingredient, and preferably only the E-type isomer represented by Formula (I-1) is used as the active ingredient;
preferably, in the pharmaceutical composition, the active ingredient is 0.1-8 parts by weight.

4. The pharmaceutical composition according to any one of claims 1-3, wherein the humectant is selected from one or more of propylene glycol, glycerol, and polyethylene glycol;
preferably, in the pharmaceutical composition, the humectant is 10-35 parts by weight;
preferably, the penetration enhancer is selected from one or more of diethylene glycol monoethyl ether, ethanol, menthol, N-methylpyrrolidone, propylene carbonate, and dimethyl isosorbide;
preferably, in the pharmaceutical composition, the penetration enhancer is 10-50 parts by weight;
preferably, the chelating agent is selected from a metal-ion-chelating agent, for example, one or more of ethylenediaminetetraacetic acid, sodium gluconate, nitrilotriacetic acid, tartaric acid, citric acid, and salts thereof;
preferably, in the pharmaceutical composition, the chelating agent is 0.01-1 part by weight;
preferably, the pH regulator is selected from a pH buffer solution, preferably a pH buffer solution of an organic acid and/or a salt thereof; for example, one or more of a citric acid/sodium citrate buffer solution, a tartrate buffer solution, an oxalate buffer solution, and an acetic acid/acetate buffer solution;
preferably, the solvent is selected from water;
preferably, in the pharmaceutical composition, the solvent is 20-60 parts by weight.

5. The pharmaceutical composition according to any one of claims 1-4, wherein the pharmaceutical composition further optionally comprises a thickener; and/or optionally comprises an antioxidant; and/or optionally comprises a preservative;
preferably, the thickener is selected from one or more of sodium carboxymethyl cellulose, hydroxypropyl methylcellulose, povidone, and polyvinyl alcohol;
preferably, in the pharmaceutical composition, the thickener is 0.01-1 part by weight;
preferably, the antioxidant is selected from one or both of propyl gallate and butylated hydroxytoluene;
preferably, in the pharmaceutical composition, the antioxidant is 0.01-0.2 parts by weight;
preferably, the preservative is selected from one or more of methyl paraben, ethyl paraben, propyl paraben, and benzalkonium chloride;
preferably, in the pharmaceutical composition, the preservative is 0.01-0.3 parts by weight;
preferably, the pharmaceutical composition has a pH of 4-6;
preferably, the pharmaceutical composition further comprises a second active ingredient.

6. The pharmaceutical composition according to any one of claims 1-5, wherein the pharmaceutical composition comprises the following components in parts by weight: 0.1-8 parts of the active ingredient, 10-35 parts of the humectant, 10-50 parts of the penetration enhancer, 0.01-1 part of the chelating agent, 0.01-0.2 parts of the antioxidant, 0.01-0.3 parts of the preservative, 20-60 parts of the solvent, and the pH regulator;
the active ingredient is the compound represented by Formula (I), preferably the aforementioned E-type isomer represented by Formula (I-1); and
preferably, the pharmaceutical composition has a pH of 4 to 6.

7. A preparation method of the pharmaceutical composition according to any one of claims 1-6, comprising mixing the components to obtain;
preferably, the preparation method comprises the following steps:
(1) mixing the active ingredient with the humectant and the penetration enhancer to form a solution containing the active ingredient;
or mixing the active ingredient, antioxidant, and preservative with the humectant and the penetration enhancer to form a solution containing the active ingredient;
(2) mixing the chelating agent and the thickener with the solvent to form a mixed solution; preferably, first dissolving the chelating agent in the solvent, and then adding the thickener for dispersion and dissolution;
(3) adding the mixed solution into the solution containing the active ingredient, mixing uniformly, and then adding the pH regulator to adjust a system pH, preferably to 4-6;
(5) adding the solvent to make up weight to obtain the pharmaceutical composition.

8. Use of the pharmaceutical composition according to any one of claims 1-6 in the preparation of a pharmaceutical formulation; preferably the pharmaceutical formulation is a solution-type formulation, for example, a spray or an aerosol;
preferably, the pharmaceutical composition or the pharmaceutical formulation is used for treating autoimmune inflammatory diseases, preferably psoriasis and/or atopic dermatitis;
preferably, the pharmaceutical composition or the pharmaceutical formulation is used for treating the above diseases occurring at hair-bearing sites, and is especially suitable for psoriasis occurring at hair-bearing sites, for example, the hair-bearing sites include, but are not limited to, scalp and axillae; preferably scalp psoriasis.

9. A pharmaceutical formulation, comprising the pharmaceutical composition according to any one of claims 1-6;
preferably, the pharmaceutical formulation is a solution-type formulation, and preferably a spray or an aerosol;
preferably, the active ingredient in the pharmaceutical formulation has a mass percentage of 0.1-8%;
preferably, the pharmaceutical formulation is used for treating autoimmune inflammatory diseases, preferably psoriasis, atopic dermatitis, and/or seborrheic dermatitis;
preferably, the pharmaceutical composition or the pharmaceutical formulation is used for treating seborrheic dermatitis or the above diseases occurring at hair-bearing sites, and is especially suitable for seborrheic dermatitis or psoriasis occurring at hair-bearing sites; preferably scalp psoriasis or seborrheic dermatitis of the scalp.

10. Use of the pharmaceutical composition according to any one of claims 1-6 in preparation of a medicament for the treatment and/or prevention of autoimmune inflammatory diseases or diseases caused by *Pityrosporum ovale;*
preferably, the autoimmune disease is preferably selected from psoriasis, atopic dermatitis, and/or seborrheic dermatitis, more preferably seborrheic dermatitis or psoriasis occurring at hair-bearing sites, and most preferably seborrheic dermatitis of the scalp or scalp psoriasis; and preferably, the diseases caused by *Pityrosporum ovale* is seborrheic dermatitis caused by *Pityrosporum ovale.*

11. A method for treating an autoimmune inflammatory disease, comprising administering to a patient an effective amount or a therapeutically effective amount of the pharmaceutical composition according to any one of claims 1-6 or the pharmaceutical formulation according to claim 9;
the autoimmune inflammatory is preferably selected from psoriasis, atopic dermatitis, and seborrheic dermatitis, and preferably, psoriasis, atopic dermatitis, and seborrheic dermatitis occurring at hair-bearing sites.

12. A method for treating and/or preventing a disease caused by *Pityrosporum ovale,* comprising administering to a patient an effective amount or a therapeutically effective amount of the pharmaceutical composition according to any one of claims 1-6 or the pharmaceutical formulation according to claim 9;
preferably, the disease caused by *Pityrosporum ovale* is seborrheic dermatitis caused by *Pityrosporum ovale.*
